# EUROPEAN PATENT APPLICATION

(11) **EP 4 559 903 A1**
(43) Date of publication of application: **28.05.2025**
(21) Application number: 23843103.5
(22) Date of filing: 24.07.2023
(51) Int. Cl.: C07D 263/57

(54) **PRODUCTION METHOD FOR TAFAMIDIS OR SALT THEREOF**

(30) Priority: 22.07.2022 JP 2022117566
(71) Applicant: Shiratori Pharmaceutical Co., Ltd., Narashino-shi, Chiba 275-0016 (JP)
(72) Inventor: YOSHINO, Hiroshi, Narashino-shi, Chiba 275-0016 (JP); SHIRO, Yuichi, Narashino-shi, Chiba 275-0016 (JP); KIMURA, Yu, Narashino-shi, Chiba 275-0016 (JP)
(74) Representative: Wächtershäuser & Hartz Patentanwaltspartnerschaft mbB
(86) International application number: PCT/JP2023/027059
(87) International publication number: WO 2024/019180

(57) **Abstract**

Provided is a method allowing the production of tafamidis or a salt thereof in a high yield. The method includes a cyclization step of cyclizing a compound of the formula (1) below in the presence of one or more copper catalysts selected from the group consisting of copper halides, copper carboxylates, copper sulfates, and copper oxides, a base, and an amine ligand to obtain a compound of the formula (2) below, 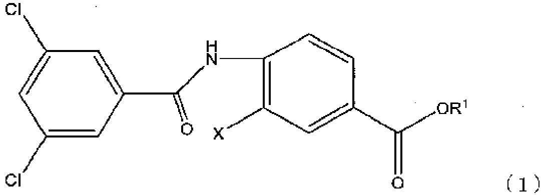 [in the formula (1), R¹ represents a linear or branched alkyl group, and X represents a halogen atom] 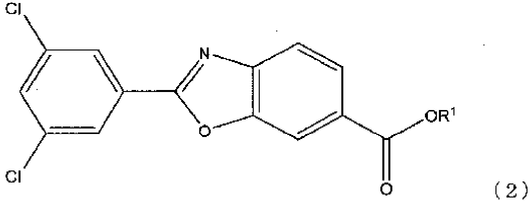 [in the formula (2), R¹ is as defined above].

## Description

### Field of the Invention

The present invention relates to a method for producing tafamidis or a salt thereof, more specifically, relates to a method for producing tafamidis or a salt thereof and a synthetic intermediate therein.

### Background of the Invention

Tafamidis meglumine is a medicine of which efficacy or effects are suppression of peripheral neuropathy progression in transthyretin-related familial amyloid polyneuropathy and transthyretin heart amyloidosis, and cardiomyopathy by transthyretin amyloidosis has been added as efficacy or an effect in recent years, and a demand therefor is predicted to increase.

As a method for producing tafamidis meglumine, for example, it is known a method of amidating 3,5-dichlorobenzoyl chloride and 3-hydroxy-4-aminobenzoic acid in the presence of pyridine and cyclizing the resulting amide compound in the presence of p-toluenesulfonic acid to obtain a benzoxazole derivative by (Patent Literatures 1 and 2).

However, the methods described in Patent Literatures 1 and 2 have a problem in that complicated purification is required because side reaction is likely to occur during amidation and reduces the selectivity and yield. In addition, there is a problem of high cost because 3-hydroxy-4-aminobenzoic acid is expensive. In particular, in the method described in Patent Literature 1, it is necessary to methyl-esterify the carboxy group derived from 3-hydroxy-4-aminobenzoic acid in order to perform purification, and column purification, which is difficult to be industrialized, is performed. In addition, since the method described in Patent Literature 1 and the method described in Patent Literature 2 require a liquid volume of 187 v/w and a liquid volume of 170 v/w, respectively, the productive efficiency is low, and since an intermediate N-[2-hydroxy-4-carboxyphenyl]-3,5-dichlorobenzamide has poor filtration properties, the industrialization is further difficult.

On the other hand, to obtain a benzoxazole derivative by cyclization (C-O coupling reaction) of N-(2-bromo-5-nitrophenyl)benzamide, it is proposed to use bis(1,5-cyclooctadiene)diiridium(I) dichloride (hereinafter, simply also referred to as iridium catalyst) as a catalyst (Non Patent Literature 1). Furthermore, this Non Patent Literature 1 discloses that cyclization reaction is performed using the iridium catalyst for 48 hours to obtain methyl 2-(3,5-dichlorophenyl)benzo[d]oxazole-6-carboxylate (methyl ester of tafamidis) in a yield of 52%.

### Citation List

### Patent Literature

Patent Literature 1: JP-A-2006-511612
Patent Literature 2: JP-A-2021-517118

### Non Patent Literature

Non Patent Literature 1: Tetrahedron Letters, 2019, 60, 151082

### Summary of the Invention

### Problem to be Solved by the Invention

In the above Non Patent Literature 1, cyclization reaction of N-(2-bromo-5-nitrophenyl)cinnamamide was performed using the iridium catalyst (0.01 mmol) and a copper iodide catalyst (CuI, 0.20 mmol), and when the iridium catalyst was used, a benzoxazole derivative was obtained in a yield of 74% without generating a by-product (debromination product).

However, when a copper iodide catalyst is used, the yield of benzoxazole derivative is only 24%, and generation of a large amount (61%) of a by-product (debromination product) was observed. Accordingly, it is considered that an iridium catalyst is more useful than a copper catalyst such as a copper iodide catalyst in order to improve the efficiency of cyclization by C-O coupling reaction.

As a result of investigation by the present inventors, it was revealed that the iridium catalyst efficiently cyclizes N-(2-bromo-5-nitrophenyl)benzamide, but there is a problem of an insufficient yield of cyclization when a synthetic intermediate of tafamidis having an alkoxycarbonylphenyl group in the molecule, such as N-[2-bromo-4-(methoxycarbonyl)phenyl]-3,5-dichlorobenzamide, is used as a substrate.

An object of the present invention is to provide a method that can produce tafamidis or a salt thereof in a high yield.

### Means for Solving the Problem

The present inventors have extensively studied cyclization reaction of N-[2-halo-4-(alkoxycarbonyl)phenyl]-3,5-dichlorobenzamide using various catalysts and as a result, found that tafamidis or a salt thereof can be surprisingly produced in a high yield and at a low cost by a method including a cyclization step of cyclizing N-[2-halo-4-(alkoxycarbonyl)phenyl]-3,5-dichlorobenzamide in the presence of one or more copper catalysts selected from the group consisting of copper halides, copper carboxylates, copper sulfates, and copper oxides, a base, and an amine ligand, to accomplish the present invention.

That is, the present invention provides the following <1> to <14>:
<1> A method for producing tafamidis or a salt thereof (hereinafter, also referred to as producing method of the present invention), comprising a cyclization step of cyclizing a compound of the formula (1) below in the presence of one or more copper catalysts selected from the group consisting of copper halides, copper carboxylates, copper sulfates, and copper oxides, a base, and an amine ligand to obtain a compound of the formula (2) below. 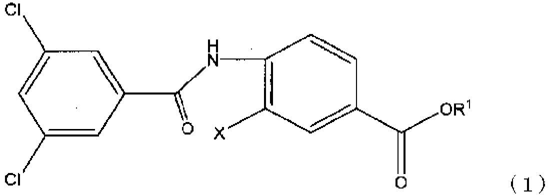 [In the formula (1),
   R¹ represents a linear or branched alkyl group, and
   X represents a halogen atom.] 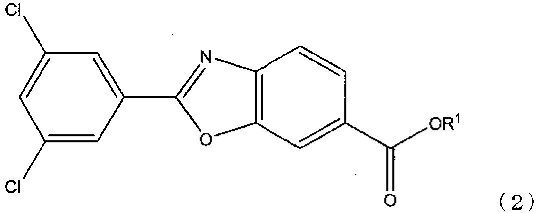
   [In the formula (2), R¹ is as defined above.]
<2> The producing method according to <1>, further comprising an amidation step of amidating a compound of the formula (3) below and a compound of the formula (4) below in the presence of a base, to use the compound obtained in this step as the compound of the formula (1) in the cyclization step. 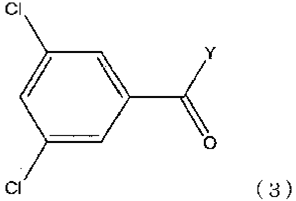 [In the formula (3), Y represents a halogen atom.] 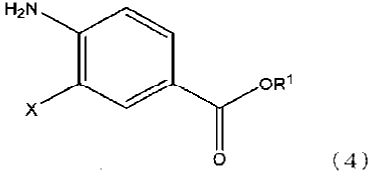 [In the formula (4), R¹ and X are as defined above.]
<3> The producing method according to <2>, further comprising a halogenation step of halogenating a compound of the formula (5) below, to use the compound obtained in this step as the compound of the formula (4) in the amidation step. 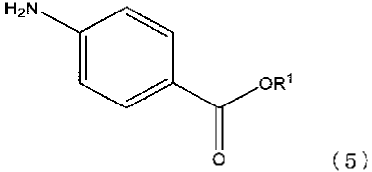 [In the formula (5), R¹ is as defined above.]
<4> The producing method according to <2>, further comprising an esterification step of subjecting a compound of the formula (11) below and a compound of the formula (12) below to dehydration-condensation reaction, to use the compound obtained in this step as the compound of the formula (4) in the amidation step. 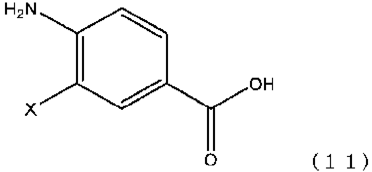 [In the formula (11), X is as defined above.]

   R¹OH (12)

   [In the formula (12), R¹ is as defined above.]
<5> The producing method according to any one of <1> to <4>, further comprising a hydrolysis step of ester-hydrolyzing the compound of the formula (2) obtained in the cyclization step.
<6> The producing method according to any one of <1> to <5>, wherein the copper catalyst to be used is one or more selected from the group consisting of copper(I) iodide, copper(II) iodide, copper(I) bromide, copper(II) bromide, copper(I) chloride, copper(II) chloride, copper(I) acetate, copper(II) acetate, copper trifluoroacetate, copper pentafluoropropionate, copper oxalate, copper(I) sulfate, copper(II) sulfate, copper(I) oxide, and copper(II) oxide.
<7> The producing method according to any one of <1> to <5>, wherein the copper catalyst to be used is one or more copper catalysts selected from the group consisting of copper halides, copper carboxylates, and copper sulfates.
<8> The producing method according to any one of <1> to <5>, wherein the copper catalyst to be used is a copper halide.
<9> The producing method according to <8>, wherein the copper halide to be used is one or more copper halides selected from the group consisting of copper(I) iodide, copper(I) bromide, and copper(I) chloride.
<10> The producing method according to any one of <1> to <9>, wherein the amine ligand to be used is an amine ligand selected from the group consisting of monovalent amine ligands and diamine ligands.
<11> The producing method according to any one of <1> to <10>, wherein X is a chlorine atom or a bromine atom.
<12> A compound of the formula (1) below (hereinafter, also referred to as specific compound of the present invention). 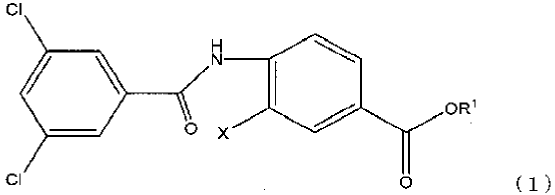 [In the formula (1),
   R¹ represents a linear or branched alkyl group, and
   X represents a fluorine atom, a bromine atom, or an iodine atom.]
<13> The compound according to <12>, wherein R¹ is an ethyl group.
<14> The compound according to <12> or <13>, wherein X is a bromine atom or an iodine atom.

### Advantageous Effects of Invention

According to the present invention, tafamidis or a salt thereof can be produced in a high yield and at a low cost.

The specific compound of the present invention is useful as a synthetic intermediate of tafamidis or a salt thereof.

### Detailed Description of the Invention

A producing method of the present invention is characterized in including a cyclization step of cyclizing a compound of the formula (1) below (hereinafter, also referred to as compound (1)) in the presence of one or more copper catalysts selected from the group consisting of copper halides, copper carboxylates, copper sulfates, and copper oxides, a base, and an amine ligand, to obtain a compound of the formula (2) below (hereinafter, also referred to as compound (2)). Since the cyclization step in the producing method of the present invention is unlikely to cause side reaction and can produce quantitatively the compound (2) with a high purity, the producing method according to the present invention is an industrially excellent producing method. In the cyclization step in the producing method of the present invention, the necessary liquid volume (the maximum liquid volume before separation of the product) is small, and it is easy to reduce the size of the production facilities necessary for producing tafamidis. 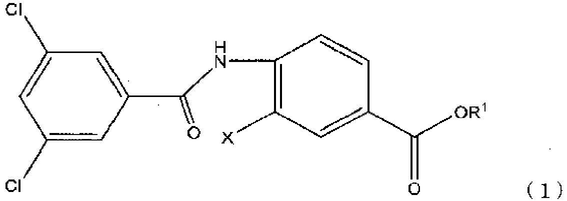 [In the formula (1),
R¹ represents a linear or branched alkyl group, and
X represents a halogen atom.] 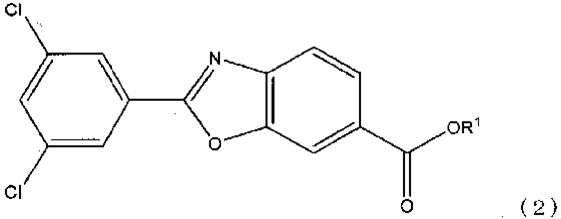
[In the formula (2), R¹ is as defined above.]

### (Cyclization step)

In the formula (1), R¹ represents a linear or branched alkyl group. From the viewpoint of availability and reaction efficiency, the alkyl group preferably has 1 to 12, more preferably 1 to 8, further more preferably 1 to 4, and particularly preferably 1 or 2 carbon atoms. Examples of the alkyl group include a methyl group, an ethyl group, an n-propyl group, an isopropyl group, an n-butyl group, an isobutyl group, a sec-butyl group, and a tert-butyl group. Among these groups, a methyl group and an ethyl group are preferable, and an ethyl group is more preferable.

Examples of the halogen atom of X include a fluorine atom, a chlorine atom, a bromine atom, and an iodine atom. Among these atoms, from the viewpoint of reaction efficiency and by-product suppression, a chlorine atom, a bromine atom, and an iodine atom are preferable, and a chlorine atom and a bromine atom are more preferable.

Examples of the compound (1) include N-[2-bromo-4-(methoxycarbonyl)phenyl]-3,5-dichlorobenzamide, N-[2-bromo-4-(ethoxycarbonyl)phenyl]-3,5-dichlorobenzamide, N-[2-bromo-4-(n-propoxycarbonyl)phenyl]-3,5-dichlorobenzamide, N-[2-bromo-4-(isopropoxycarbonyl)phenyl]-3,5-dichlorobenzamide, N-[2-chloro-4-(methoxycarbonyl)phenyl]-3,5-dichlorobenzamide, N-[2-chloro-4-(ethoxycarbonyl)phenyl]-3,5-dichlorobenzamide, N-[2-chloro-4-(n-propoxycarbonyl)phenyl]-3,5-dichlorobenzamide, N-[2-chloro-4-(isopropoxycarbonyl)phenyl]-3,5-dichlorobenzamide, N-[2-iodo-4-(methoxycarbonyl)phenyl]-3,5-dichlorobenzamide, N-[2-iodo-4-(ethoxycarbonyl)phenyl]-3,5-dichlorobenzamide, N-[2-iodo-4-(n-propoxycarbonyl)phenyl]-3,5-dichlorobenzamide, and N-[2-iodo-4-(isopropoxycarbonyl)phenyl]-3,5-dichlorobenzamide.

The cyclization step uses one or more copper catalysts selected from the group consisting of copper halides, copper carboxylates, copper sulfates, and copper oxides. The use of this copper catalyst can proceed the cyclization reaction with high efficiency. Among these catalysts, from the viewpoint of reaction efficiency and by-product suppression, one or more copper catalysts selected from the group consisting of copper halides, copper carboxylates, and copper sulfates are preferable.

The copper catalyst is, from the viewpoint of reaction efficiency and by-product suppression, preferably one or more selected from the group consisting of copper(I) iodide, copper(II) iodide, copper(I) bromide, copper(II) bromide, copper(I) chloride, copper(II) chloride, copper(I) acetate, copper(II) acetate, copper trifluoroacetate, copper pentafluoropropionate, copper oxalate, copper(I) sulfate, copper(II) sulfate, copper(I) oxide, and copper(II) oxide; more preferably one or more selected from the group consisting of copper(I) iodide, copper(II) iodide, copper(I) bromide, copper(II) bromide, copper(I) chloride, copper(II) chloride, copper(I) acetate, copper(II) acetate, copper(I) sulfate, copper(II) sulfate, copper(I) oxide, and copper(II) oxide; further more preferably one or more selected from the group consisting of copper(I) iodide, copper(I) bromide, copper(I) chloride, copper(I) acetate, copper(II) sulfate, and copper(II) oxide; further more preferably one or more selected from the group consisting of copper(I) iodide, copper(I) bromide, copper(I) chloride, copper acetate, and copper(II) sulfate; and particularly preferably copper(I) iodide.

The amount of one or more copper catalysts selected from the group consisting of copper halides, copper carboxylates, copper sulfates, and copper oxides to be used is usually in a range of from 0.1 to 500 moles with respect to 100 moles of the compound (1) and is, from the viewpoint of reaction efficiency and manufacturing cost, preferably in a range of from 0.5 to 100 moles, and more preferably in a range of from 1 to 50 moles.

The base that is used in the cyclization step is preferably an inorganic base. Examples of the base include alkali metal carbonates such as potassium carbonate, sodium carbonate, and lithium carbonate; alkali metal hydrogen carbonates such as potassium hydrogen carbonate, sodium hydrogen carbonate, and lithium hydrogen carbonate; and alkali metal hydroxides such as sodium hydroxide and potassium hydroxide. The bases may be used alone or in combination of two or more.

The amount of the base to be used in the cyclization step is, from the viewpoint of reaction efficiency and manufacturing cost, preferably in a range of from 10 to 1,000 moles with respect to 100 moles of the compound (1), more preferably in a range of from 100 to 750 moles, and particularly preferably in a range of from 200 to 500 moles.

Examples of the amine ligand that is used in the cyclization step include monovalent amine ligands such as ethanolamine; and diamine ligands such as tetramethylethylene diamine, N,N'-dimethylethylene diamine, N,N'-dicyclohexylethylene diamine, 2,2'-bipyridine, 1,10-phenanthroline, 2,9-diphenyl-1,10-phenanthroline, ethylene diamine, N,N,N',N'-tetramethyl-1,3-propane diamine, 2,2'-bi[2-oxazoline], 1,2-cyclohexane diamine, N,N'-ditert-butylethylene diamine, ethylenebis(diethylamine), and N,N'-diphenylethylene diamine. Among the monovalent amine ligands and diamine ligands, from the viewpoint of reaction efficiency and manufacturing cost, a diamine ligand is preferable. The amine ligands may be used alone or in combination of two or more.

Among these amine ligands, from the viewpoint of reaction efficiency and manufacturing cost, preferred are tetramethylethylene diamine, N,N'-dimethylethylene diamine, N,N'-dicyclohexylethylene diamine, 2,2'-bipyridine, 1,10-phenanthroline, 2,9-diphenyl-1,10-phenanthroline, ethylene diamine, ethanolamine, N,N,N',N'-tetramethyl-1,3-propane diamine, 2,2'-bi[2-oxazoline], and 1,2-cyclohexane diamine; and more preferred are tetramethylethylene diamine, N,N'-dimethylethylene diamine, N,N'-dicyclohexylethylene diamine, 2,2'-bipyridine, 1,10-phenanthroline, and 2,9-diphenyl-1,10-phenanthroline.

The amount of the amine ligand to be used in the cyclization step is, from the viewpoint of reaction efficiency and manufacturing cost, preferably in a range of from 0.1 to 1,000 moles with respect to 100 moles of the compound (1), more preferably in a range of from 0.5 to 200 moles, and particularly preferably in a range of from 1 to 100 moles.

The cyclization step is preferably performed in the presence of a solvent, more preferably in the presence of an organic solvent, from the viewpoint of reaction efficiency and manufacturing cost.

Examples of the organic solvent include aromatic hydrocarbon solvents such as benzene, toluene, and xylene; aliphatic hydrocarbon solvents such as propane, n-butane, isobutane, n-pentane, isopentane, and n-hexane; halogenated hydrocarbon solvents such as methylene chloride, ethylene chloride, dichloromethane, and chloroform; ether solvents such as diethyl ether, dibutyl ether, tert-butyl methyl ether, dimethoxyethane, tetrahydrofuran, and dioxane; ester solvents such as ethyl acetate; and other solvents such as N,N-dimethylformamide, N,N-dimethylacetamide, and dimethyl sulfoxide. The solvents may be used alone or in combination of two or more.

The amount of the solvent to be used is preferably in a range of from 150 to 5,000 parts by mass with respect to 100 parts by mass of the compound (1) and more preferably in a range of from 300 to 3,000 parts by mass.

The reaction temperature of the cyclization step is usually from ordinary temperature to the solvent boiling point and preferably from 50°C to the solvent boiling point.

The reaction period of time of the cyclization step is usually from 0.1 to 72 hours and preferably 1 to 48 hours.

The cyclization step can be performed by a batch method, a semi-continuous method, or a continuous method.

The compound (1) is preferably obtained by an amidation step of amidating a compound of the formula (3) below (hereinafter, also referred to as compound (3)) and a compound of the formula (4) below (hereinafter, also referred to as compound (4)) in the presence of a base. The amidation step quantitatively proeeds and can suppress the necessary liquid volume (the maximum liquid volume before separation of the product), and also the aftertreatment is simple. Thus, it is possible to further increase the yield and reduce the cost by obtaining the compound (1) through the amidation step.

The compound (4) can be obtained by, for example, a halogenation step of halogenating a compound of the formula (5) below (hereinafter, also referred to as compound (5)) or an esterification step of subjecting a compound of the formula (11) below (hereinafter, also referred to as compound (11)) and a compound of the formula (12) below (hereinafter, also referred to as compound (12)) to dehydration-condensation reaction.

The halogenation step quantitatively proceeds and can suppress the necessary liquid volume (the maximum liquid volume before separation of the product), and also the aftertreatment is simple. It is possible to further increase the yield and reduce the cost by obtaining the compound (4) through the halogenation step. The compound (3) to be used may be a commercially available product or may be prepared with reference to a known method. 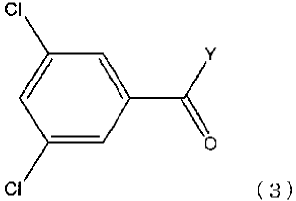 [In the formula (3), Y represents a halogen atom.] 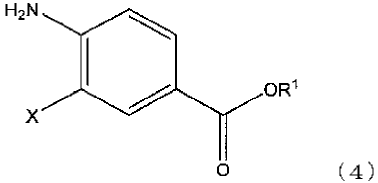 [In the formula (4), R¹ and X are as defined above.] 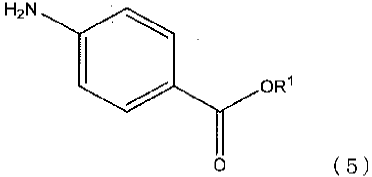 [In the formula (5), R¹ is as defined above.] 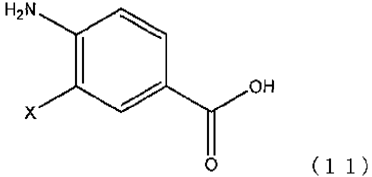 [In the formula (11), X is as defined above.]

R¹OH (12)

[In the formula (12), R¹ is as defined above.]

Here, the halogenation step, the esterification step, and the amidation step will be described in detail.

### (Halogenation step)

Examples of the compound (5) include methyl 4-aminobenzoate, ethyl 4-aminobenzoate, n-propyl 4-aminobenzoate, and isopropyl 4-aminobenzoate. The compound (5) is available inexpensively, and it is possible to drastically reduce the cost by obtaining the compound (4) through the halogenation step.

The halogenation step is preferably performed in the presence of a halogenating agent.

Examples of the halogenating agent include brominating agents such as bromine (Br₂), hydrogen bromide, N-bromosuccinimide, tetrabutylammonium tribromide, dibromoisocyanuric acid (DBI), and 1,3-dibromo-5,5-dimethylhydantoin; chlorinating agents such as chlorine (Cl₂), thionyl chloride, and N-chlorosuccinimide; fluorinating agents such as fluorine (F₂); and iodinating agents such as iodine (I₂), iodine chloride (ICl), 1,3-diiodo-5,5-dimethylhydantoin, and N-iodosuccinimide. The halogenating agents may be used alone or in combination of two or more.

The amount of the halogenating agent to be used is, from the viewpoint of reaction efficiency and manufacturing cost, preferably in a range of from 80 to 120 moles with respect to 100 moles of the compound (5), more preferably in a range of from 90 to 110 moles, and particularly preferably in a range of from 95 to 105 moles.

The halogenation step is preferably performed in the presence of a solvent, more preferably in the presence of an organic solvent, from the viewpoint of reaction efficiency and manufacturing cost.

Examples of the organic solvent include ether solvents such as diethyl ether, dibutyl ether, tert-butyl methyl ether, dimethoxyethane, tetrahydrofuran, and dioxane; amide solvents such as N,N-dimethylformamide; halogenated hydrocarbon solvents such as methylene chloride, ethylene chloride, dichloromethane, and chloroform; nitrile solvents such as acetonitrile; and monovalent lower alcohol solvents such as methanol and ethanol. The solvents may be used alone or in combination of two or more.

The amount of the solvent to be used is preferably in a range of from 100 to 2,000 parts by mass with respect to 100 parts by mass of the compound (5) and more preferably in a range of from 250 to 1,000 parts by mass.

The reaction temperature of the halogenation step is usually from -15°C to the solvent boiling point and preferably from 0°C to 50°C.

The reaction period of time of the halogenation step is usually from 0.1 to 24 hours and preferably from 0.5 to 12 hours.

### (Esterification step)

Examples of the compound (11) include 4-amino-3-chlorobenzoic acid, 4-amino-3-bromobenzoic acid, 4-amino-3-iodobenzoic acid, and 4-amino-3-fluorobenzoic acid.

Examples of the compound (12) include monovalent lower alcohols (specifically, monovalent alcohols having 1 to 4 carbon atoms) such as methanol, ethanol, n-propanol, and isopropanol.

The amount of the compound (12) to be used is, from the viewpoint of reaction efficiency and manufacturing cost, preferably in a range of from 90 to 50,000 moles with respect to 100 moles of the compound (11) and more preferably in a range of from 100 to 10,000 moles.

The esterification step is preferably performed in the presence of an esterification catalyst.

Examples of the esterification catalyst include inorganic acids such as sulfuric acid and phosphoric acid; inorganic oxides such as tin oxide and zinc oxide; and alcoholate. The esterification catalysts may be used alone or in combination of two or more.

The amount of the esterification catalyst to be used in the esterification step is, from the viewpoint of reaction efficiency and manufacturing cost, preferably in a range of from 10 to 1,000 moles with respect to 100 moles of the compound (11) and more preferably in a range of from 50 to 300 moles.

Since the compound (12) acts not only as a substrate but also as a solvent, the esterification step can be performed even not using a solvent other than the compound (12), but a solvent other than the compound (12) may be further used together with the compound (12). Examples of such a solvent other than the compound (12) include ether solvents, halogenated hydrocarbon solvents, and nitrile solvents, as in the halogenation step.

The reaction temperature of the esterification step is usually from 35°C to the solvent boiling point.

The reaction period of time of the esterification step is usually from 0.1 to 36 hours and preferably from 0.5 to 12 hours.

### (Amidation step)

Examples of the compound (4) include methyl 4-amino-3-chlorobenzoate, methyl 4-amino-3-bromobenzoate, methyl 4-amino-3-iodobenzoate, ethyl 4-amino-3-chlorobenzoate, ethyl 4-amino-3-bromobenzoate, ethyl 4-amino-3-iodobenzoate, n-propyl 4-amino-3-chlorobenzoate, n-propyl 4-amino-3-bromobenzoate, n-propyl 4-amino-3-iodobenzoate, isopropyl 4-amino-3-chlorobenzoate, isopropyl 4-amino-3-bromobenzoate, and isopropyl 4-amino-3-iodobenzoate.

Examples of the halogen atom of Y include a fluorine atom, a chlorine atom, a bromine atom, and an iodine atom. Among these atoms, from the viewpoint of reaction efficiency and by-product suppression, a chlorine atom, a bromine atom, and an iodine atom are preferable, and a chlorine atom and a bromine atom are more preferable.

Examples of the compound (3) include 3,5-dichlorobenzoyl chloride, 3,5-dichlorobenzoyl bromide, and 3,5-dichlorobenzoyl iodide.

The amount of the compound (3) to be used is, from the viewpoint of reaction efficiency and manufacturing cost, preferably in a range of from 70 to 1,000 moles with respect to 100 moles of the compound (4), more preferably in a range of from 90 to 500 moles, and particularly preferably in a range of from 100 to 200 moles.

Examples of the base that is used in the amidation step include, in addition to tertiary amine bases, alkali metal carbonates such as potassium carbonate and sodium carbonate; and alkali metal hydroxides such as potassium hydroxide and sodium hydroxide. In particular, a tertiary amine base is preferable. Examples of such a base include pyridine, 4-dimethylaminopyridine, triethylamine, diisopropylethylamine, and N-methylmorpholine. The bases may be used alone or in combination of two or more.

The amount of the base to be used in the amidation step is, from the viewpoint of reaction efficiency and manufacturing cost, preferably in a range of from 70 to 1,000 moles with respect to 100 moles of the compound (4), more preferably in a range of from 90 to 500 moles, and particularly preferably in a range of from 100 to 200 moles.

The amidation step is preferably performed in the presence of a solvent, more preferably in the presence of an organic solvent, from the viewpoint of reaction efficiency and manufacturing cost.

Examples of the organic solvent include ether solvents such as diethyl ether, dibutyl ether, tert-butyl methyl ether, dimethoxyethane, tetrahydrofuran, and dioxane; halogenated hydrocarbon solvents such as methylene chloride, ethylene chloride, dichloromethane, and chloroform; nitrile solvents such as acetonitrile; ester solvents such as ethyl acetate; and aromatic hydrocarbon solvents such as benzene, toluene, and xylene. The solvents may be used alone or in combination of two or more.

The amount of the solvent to be used is preferably in a range of from 150 to 3,000 parts by mass with respect to 100 parts by mass of the compound (4) and more preferably in a range of from 300 to 1,000 parts by mass.

The reaction temperature of the amidation step is usually from 3°C to the solvent boiling point and preferably from 10°C to 30°C.

The reaction period of time of the amidation step is usually from 0.1 to 36 hours and preferably 0.5 to 12 hours.

Among the compounds (1) obtained in this amidation step, a compound of which X is a fluorine atom, a bromine atom, or an iodine atom (preferably a bromine atom or an iodine atom) is a new compound. This compound is useful as a synthetic intermediate of tafamidis or a salt thereof.

R¹ in the formula (1) is as defined above and is preferably a methyl group or an ethyl group and more preferably an ethyl group.

The producing method of the present invention preferably further includes a hydrolysis step of ester-hydrolyzing the compound (2) obtained in the cyclization step. Tafamidis of the formula (6) below is obtained by performing such a hydrolysis step.

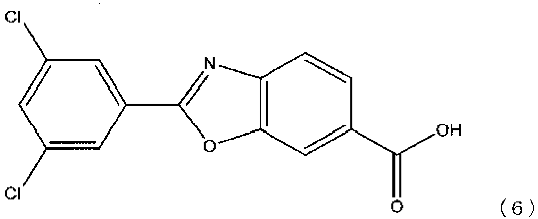

### (Hydrolysis step)

The hydrolysis is preferably performed in the presence of a base.

The base that is used in the hydrolysis step is preferably an inorganic base such as lithium hydroxide, sodium hydroxide, potassium hydroxide, calcium hydroxide, and cesium hydroxide. The bases may be used alone or in combination of two or more.

The amount of the base to be used in the hydrolysis step is, from the viewpoint of reaction efficiency and manufacturing cost, preferably in a range of from 70 to 750 moles with respect of 100 moles of the compound (2), more preferably in a range of from 90 to 500 moles, and particularly preferably in a range of from 100 to 200 moles.

The amount of water to be used in the hydrolysis step is, from the viewpoint of reaction efficiency and manufacturing cost, preferably in a range of from 75 to 2,000 parts by mass with respect to 100 parts by mass of the compound (2) and more preferably in a range of from 100 to 800 parts by mass.

An organic solvent that is miscible with water may be used together with water. Examples of the organic solvent include ether solvents such as diethyl ether, dibutyl ether, tert-butyl methyl ether, dimethoxyethane, tetrahydrofuran, and dioxane; nitrile solvents such as acetonitrile; and monovalent lower alcohol solvents such as methanol and ethanol. The solvents may be used alone or in combination of two or more.

The reaction temperature of the hydrolysis step is usually from 10°C to the solvent boiling point and preferably from 20°C to 70°C.

The reaction period of time of the hydrolysis step is usually from 1 to 96 hours and preferably from 3 to 48 hours.

A salt of tafamidis can be obtained according to a usual method, for example, by a salification step of bringing the tafamidis (6) into contact with an inorganic base such as lithium hydroxide, sodium hydroxide, potassium hydroxide, calcium hydroxide, or cesium hydroxide; or an organic base such as methylamine, ethylamine, meglumine, ethanolamine, diethanolamine, dicyclohexylamine, or benzylamine.

The amount of the base to be used in the salification step is, from the viewpoint of reaction efficiency and manufacturing cost, preferably in a range of from 70 to 750 moles with respect to 100 moles of the tafamidis (6), more preferably in a range of from 90 to 500 moles, and particularly preferably in a range of from 100 to 300 moles.

The salification step is preferably performed in the presence of a solvent. Examples of the solvent include ether solvents such as diethyl ether, dibutyl ether, tert-butyl methyl ether, dimethoxyethane, tetrahydrofuran, and dioxane; nitrile solvents such as acetonitrile; ester solvents such as ethyl acetate; monovalent lower alcohol solvents such as methanol and ethanol; and water. The solvents may be used alone or in combination of two or more.

The reaction temperature of the salification step is usually from -10°C to the solvent boiling point and preferably from 0°C to 30°C.

The reaction period of time of the salification step is usually from 0.1 to 96 hours and preferably from 1 to 48 hours.

The tafamidis (6) obtained in the above-described hydrolysis step may be subjected to the salification step after isolation and purification, or the solution of tafamidis (6) obtained in the hydrolysis step may be directly subjected to the salification step.

The reaction product obtained in each of the above steps may be purified by a separation method such as distillation, extraction, crystallization, and washing.

According to the producing method of the present invention, tafamidis or a salt thereof can be produced in a high yield and at a low cost. The necessary liquid volume (the maximum liquid volume before separation of the product) in the cyclization step is small, and it is easy to reduce the size of the production facilities necessary for producing tafamidis.

Since the necessary liquid volumes (the maximum liquid volumes before separation of the products) in the above amidation step and the above halogenation step are also small, it is easy to reduce the size of the production facilities necessary for producing tafamidis, even when the producing method of the present invention includes the amidation step and/or the halogenation step.

### Examples

The present invention will hereinafter be described in detail with reference to Examples, but is not limited to these Examples.

### Example 1 (Synthesis of tafamidis)

### (1) Synthesis of ethyl 4-amino-3-bromobenzoate

Ethyl 4-aminobenzoate (200 g, 1.21 mol) was dissolved in tetrahydrofuran (1,000 mL), followed by stirring at an outside temperature of 0°C. Subsequently, N-bromosuccinimide (218 g, 1.22 mol) was added thereto, followed by stirring for 1 hour. To this reaction solution, water (1,000 mL) and ethyl acetate (1,000 mL) were added for liquid separation. The organic layer was washed with a saturated saline (1,000 mL), then dehydrated over anhydrous sodium sulfate, and concentrated under reduced pressure. Ethyl acetate (460 mL) and n-heptane (1,500 mL) were added to the concentrate, followed by stirring at an outside temperature of 0°C for 1 hour. The precipitated solid was then collected by filtration. The solid collected by filtration was dried under reduced pressure at an outside temperature of 40°C to obtain ethyl 4-amino-3-bromobenzoate (297 g, 1.22 mol, yield: 100%) as a slightly yellowish white solid.
¹H-NMR (DMSO): δ/ppm = 1.28 (t, 3H, J = 7.2 Hz), 4.22 (q, 2H, J = 6.8 Hz), 6.19 (s, 2H), 6.80 (d, 1H, J = 8.4 Hz), 7.65 (dd, 1H, J = 2.0, 8.4 Hz), 7.89 (d, 1H, J = 2.0 Hz)

### (2) Synthesis of N-[2-bromo-4-(ethoxycarbonyl)phenyl]-3,5-dichlorobenzamide

The ethyl 4-amino-3-bromobenzoate (290 g, 1.19 mol) obtained above was dissolved in tetrahydrofuran (2,900 mL), and pyridine (141 g, 1.79 mol) was added thereto. Subsequently, 3,5-dichlorobenzoyl chloride (299 g, 1.42 mol) dissolved in tetrahydrofuran (580 mL) was dropwise added thereto, followed by stirring at an outside temperature of 15°C for 19.5 hours. Water (3,480 mL) was added to the reaction solution, followed by stirring at an outside temperature of 0°C for 1 hour. The precipitated solid was then collected by filtration. The solid collected by filtration was dried under reduced pressure at an outside temperature of 50°C to obtain N-[2-bromo-4-(ethoxycarbonyl)phenyl]-3,5-dichlorobenzamide (460 g, 1.10 mol, yield: 93%) as a white solid.
¹H-NMR (DMSO): δ/ppm = 1.34 (t, 3H, J = 7.2 Hz), 4.34 (q, 2H, J = 7.2 Hz), 7.75 (d, 1H, J = 8.4 Hz), 7.93 (t, 1H, J = 1.6 Hz), 8.01 (dd, 1H, J = 1.6, 7.6 Hz), 8.01 (d, 2H, J = 2.0 Hz), 8.21 (1H, d, J = 1.6 Hz), 10.48 (1H, s)

### (3) Synthesis of ethyl 2-(3,5-dichlorophenyl)-1,3-benzoxazole-6-carboxylate

N,N,N',N'-Tetramethylethylene diamine (0.28 g, 2.4 mmol), copper(I) iodide (0.23 g, 1.2 mmol), N-[2-bromo-4-(ethoxycarbonyl)phenyl]-3,5-dichlorobenzamide (5.00 g, 12.0 mmol) obtained above, and potassium carbonate (4.97 g, 36.0 mmol) were added to toluene (50 mL), followed by stirring at an outside temperature of 110°C for 5 hours. To this reaction solution, a 10% ammonium chloride aqueous solution (25 mL) and tetrahydrofuran (60 mL) were added for liquid separation. The organic layer was washed with a 10% ammonium chloride aqueous solution and a saturated saline solution, then dehydrated over anhydrous sodium sulfate, and concentrated under reduced pressure to obtain ethyl 2-(3,5-dichlorophenyl)-1,3-benzoxazole-6-carboxylate (3.99 g, 11.9 mmol, yield: 99%) as a light yellow solid.
¹H-NMR (DMSO): δ/ppm = 1.44 (t, 3H, J = 7.2 Hz), 4.43 (q, 2H, J = 7.2 Hz), 7.55 (t, 1H, J = 2.0), 7.80 (d, 1H, J = 8.4 Hz), 8.13 (dd, 1H, J = 2.0, 8.8 Hz), 8.15 (d, 1H, J = 2.0 Hz), 8.29 (d, 1H, J = 1.2 Hz)

### (4) Synthesis of tafamidis

Ethyl 2-(3,5-dichlorophenyl)-1,3-benzoxazole-6-carboxylate (3.99 g, 11.9 mmol) obtained above and lithium hydroxide monohydrate (0.70 g, 16.6 mmol) were added to a mixture solution of tetrahydrofuran (64 mL) and water (16 mL), followed by stirring overnight at an outside temperature of 40°C. The reaction solution was cooled, and water (48 mL) and 1 mol/L hydrochloric acid (32 mL) were added thereto. The precipitated solid was collected by filtration. The solid collected by filtration was dried under reduced pressure at an outside temperature of 50°C to obtain tafamidis (3.17 g, 10.3 mmol, yield: 87%, total yield from ethyl 4-aminobenzoate: 80%) as a slightly yellowish white solid.
¹H-NMR (DMSO): δ/ppm = 7.91 (d, 1H, J = 8.4 Hz), 7.94 (t, 1H, J = 2.0 Hz), 8.03 (dd, 1H, 1.2,8.0 Hz), 8.13 (d, 2H, J = 2.0 Hz), 8.26 (d, 1H, J = 0.8 Hz), 13.28 (br-s, 1H)

### Example 2 (Synthesis of tafamidis)

N,N,N',N'-Tetramethylethylene diamine (0.28 g, 2.4 mmol), copper(I) bromide (0.17 g, 1.2 mmol), N-[2-bromo-4-(ethoxycarbonyl)phenyl]-3,5-dichlorobenzamide (5.00 g, 12.0 mmol) obtained by the same procedures as in (1) and (2) of Example 1, and potassium carbonate (4.97 g, 36.0 mmol) were added to toluene (50 mL), followed by stirring overnight at an outside temperature of 110°C. To this reaction solution, a 10% ammonium chloride aqueous solution (25 mL) and tetrahydrofuran (60 mL) were added for liquid separation. The organic layer was washed with a 10% ammonium chloride aqueous solution and a saturated saline, then dehydrated over anhydrous sodium sulfate, and concentrated under reduced pressure to obtain ethyl 2-(3,5-dichlorophenyl)-1,3-benzoxazole-6-carboxylate (3.88 g, 11.5 mmol, yield: 96%) as a light yellow solid. The obtained spectral data were consistent with the spectral data described in (3) of Example 1.

Subsequently, the same procedure as in (4) of Example 1 was performed to obtain tafamidis in a yield of 87% (total yield from ethyl 4-aminobenzoate: 78%) as a slightly yellowish white solid. The obtained spectral data were consistent with the spectral data described in (4) of Example 1.

### Example 3 (Synthesis of tafamidis)

N,N,N',N'-Tetramethylethylene diamine (0.28 g, 2.4 mmol), copper(I) acetate (0.15 g, 1.2 mmol), N-[2-bromo-4-(ethoxycarbonyl)phenyl]-3,5-dichlorobenzamide (5.00 g, 12.0 mmol) obtained by the same procedures as in (1) and (2) of Example 1, and potassium carbonate (4.97 g, 36.0 mmol) were added to toluene (50 mL), followed by stirring at an outside temperature of 110°C for 5 hours. To this reaction solution, a 10% ammonium chloride aqueous solution (25 mL) and tetrahydrofuran (60 mL) were added for liquid separation. The organic layer was washed with a 10% ammonium chloride aqueous solution and a saturated saline, then dehydrated over anhydrous sodium sulfate, and concentrated under reduced pressure to obtain ethyl 2-(3,5-dichlorophenyl)-1,3-benzoxazole-6-carboxylate (3.89 g, 11.6 mmol, yield: 96%) as a grayish yellow solid. The obtained spectral data were consistent with the spectral data described in (3) of Example 1.

Subsequently, the same procedure as in (4) of Example 1 was performed to obtain tafamidis in a yield of 87% (total yield from ethyl 4-aminobenzoate: 78%) as a grayish white solid. The obtained spectral data were consistent with the spectral data described in (4) of Example 1.

### Example 4 (Synthesis of tafamidis)

N,N,N',N'-Tetramethylethylene diamine (0.28 g, 2.4 mmol), copper(II) sulfate (0.19 g, 1.2 mmol), N-[2-bromo-4-(ethoxycarbonyl)phenyl]-3,5-dichlorobenzamide (5.00 g, 12.0 mmol) obtained by the same procedures as in (1) and (2) of Example 1, and potassium carbonate (4.97 g, 36.0 mmol) were added to toluene (50 mL), followed by stirring at an outside temperature of 110°C for 5 hours. To this reaction solution, a 10% ammonium chloride aqueous solution (25 mL) and tetrahydrofuran (60 mL) were added for liquid separation. The organic layer was washed with a 10% ammonium chloride aqueous solution and a saturated saline, then dehydrated over anhydrous sodium sulfate, and concentrated under reduced pressure to obtain ethyl 2-(3,5-dichlorophenyl)-1,3-benzoxazole-6-carboxylate (3.89 g, 11.6 mmol, yield: 96%) as a light yellow solid. The obtained spectral data were consistent with the spectral data described in (3) of Example 1.

Subsequently, the same procedure as in (4) of Example 1 was performed to obtain tafamidis in a yield of 90% (total yield from ethyl 4-aminobenzoate: 80%) as a slightly yellowish white solid. The obtained spectral data were consistent with the spectral data described in (4) of Example 1.

### Example 5 (Synthesis of tafamidis)

N,N,N',N'-Tetramethylethylene diamine (0.28 g, 2.4 mmol), copper(II) oxide (0.10 g, 1.2 mmol), N-[2-bromo-4-(ethoxycarbonyl)phenyl]-3,5-dichlorobenzamide (5.00 g, 12.0 mmol) obtained by the same procedures as in (1) and (2) of Example 1, and potassium carbonate (4.97 g, 36.0 mmol) were added to toluene (50 mL), followed by stirring at an outside temperature of 110°C for 24 hours. The reaction conversion rate on this occasion was 93%. To this reaction solution, a 10% ammonium chloride aqueous solution (25 mL) and tetrahydrofuran (60 mL) were added for liquid separation. The organic layer was washed with a 10% ammonium chloride aqueous solution and a saturated saline, then dehydrated over anhydrous sodium sulfate, and concentrated under reduced pressure. Ethyl acetate (100 mL) was added to the concentrate to dissolve the concentrate at an outside temperature of 80°C, followed by cooling to room temperature. The precipitated crystals were collected by filtration. The crystals were dried under reduced pressure at an outside temperature of 50°C to obtain ethyl 2-(3,5-dichlorophenyl)-1,3-benzoxazole-6-carboxylate (2.87 g, 8.5 mmol, yield: 71%) as a white solid.

Subsequently, the same procedure as in (4) of Example 1 was performed to obtain tafamidis in a yield of 97% (total yield from ethyl 4-aminobenzoate: 64%) as a slightly yellowish white solid. The obtained spectral data were consistent with the spectral data described in (4) of Example 1.

### Example 6 (Synthesis of tafamidis)

N-[2-Chloro-4-(ethoxycarbonyl)phenyl]-3,5-dichlorobenzamide was obtained by performing the same procedures as in (1) and (2) of Example 1 except that N-bromosuccinimide was changed to N-chlorosuccinimide.

N,N,N',N'-Tetramethylethylene diamine (0.19 g, 1.61 mmol), copper(I) acetate (0.10 g, 0.8 mmol), N-[2-chloro-4-(ethoxycarbonyl)phenyl]-3,5-dichlorobenzamide (1.00 g, 2.7 mmol) obtained above, and potassium carbonate (1.11 g, 8.1 mmol) were added to toluene (10 mL), followed by stirring at an outside temperature of 110°C for 22 hours. To this reaction solution, a 10% ammonium chloride aqueous solution (5 mL) and tetrahydrofuran (12 mL) were added for liquid separation. The organic layer was washed with a 10% ammonium chloride aqueous solution and a saturated saline, then dehydrated over anhydrous sodium sulfate, and concentrated under reduced pressure to obtain ethyl 2-(3,5-dichlorophenyl)-1,3-benzoxazole-6-carboxylate (0.91 g, 2.7 mmol, yield: 100%) as a light yellow solid. The obtained spectral data were consistent with the spectral data described in (3) of Example 1.

Subsequently, the same procedure as in (4) of Example 1 was performed to obtain tafamidis in a yield of 93% (total yield from ethyl 4-aminobenzoate: 86%) as a slightly yellowish white solid. The obtained spectral data were consistent with the spectral data described in (4) of Example 1.

### Example 7 (Synthesis of tafamidis)

### (1) Synthesis of ethyl 4-amino-3-iodobenzoate

A mixture of 4-amino-3-iodobenzoic acid (1 g, 3.8 mmol), ethanol (10 mL), and concentrated sulfuric acid (0.48 g, 4.9 mmol) was heated and refluxed for 5 hours and concentrated under reduced pressure. Saturated sodium bicarbonate water (10 mL) was added to this concentrate, and the solid was collected by filtration. The solid collected by filtration was dried under reduced pressure at an outside temperature of 50°C to obtain ethyl 4-amino-3-iodobenzoate (0.77 g, 2.6 mmol, yield: 69%) as a slightly yellowish white solid.
¹H-NMR (CDCl₃): δ/ppm = 1.38 (t, 3H, J = 7.2 Hz), 4.33 (q, 2H, J = 7.2 Hz), 4.53 (br-s, 2H), 6.72 (d, 1H, J = 8.4 Hz), 7.84 (dd, 1H, J = 2.0, 8.4 Hz), 8.35 (d, 1H, J = 1.6 Hz)

### (2) Synthesis of N-[2-iodo-4-(ethoxycarbonyl)phenyl]-3,5-dichlorobenzamide

Ethyl 4-amino-3-iodobenzoate (0.7 g, 2.4 mmol) obtained above was dissolved in tetrahydrofuran (7 mL), and pyridine (0.29 g, 3.6 mmol) was added thereto. Subsequently, 3,5-dichlorobenzoyl chloride (0.6 g, 2.9 mmol) dissolved in tetrahydrofuran (1.4 mL) was dropwise added thereto, followed by stirring at an outside temperature of 20°C for 5 hours. Ethyl acetate (11.9 mL) was added to the reaction solution for liquid separation. The organic layer was washed with a saturated saline, then dehydrated over anhydrous sodium sulfate, and concentrated under reduced pressure. Ethyl acetate (5.6 mL) was added to the concentrate, followed by stirring at room temperature for 1 hour. Then, the solid was collected by filtration. The solid collected by filtration was dried under reduced pressure at an outside temperature of 40°C to obtain N-[2-iodo-4-(ethoxycarbonyl)phenyl]-3,5-dichlorobenzamide (0.71 g, 1.5 mol, yield: 64%) as a white solid.
¹H-NMR (CDCl₃): δ/ppm = 1.34 (t, 3H, J = 7.2 Hz), 4.34 (q, 2H, J = 7.2 Hz), 7.63 (d, 1H, J = 8.4 Hz), 7.86 (d, 1H, J = 1.6 Hz), 7.93 (t, 1H, J = 1.6 Hz), 8.00-8.03 (m, 3H), 8.43 (d, 1H, J = 2.0 Hz), 8.57 (s, 1H)

### (3) Synthesis of ethyl 2-(3,5-dichlorophenyl)-1,3-benzoxazole-6-carboxylate

N,N,N',N'-Tetramethylethylene diamine (30.0 mg, 0.26 mmol), copper(I) iodide (24.6 mg, 0.13 mmol), N-[2-iodo-4-(ethoxycarbonyl)phenyl]-3,5-dichlorobenzamide (600 mg, 1.29 mmol) obtained above, and potassium carbonate (536 mg, 3.88 mmol) were added to toluene (6 mL), followed by stirring at an outside temperature of 110°C for 30 hours. To this reaction solution, a 10% ammonium chloride aqueous solution (3 mL) and tetrahydrofuran (7.2 mL) were added for liquid separation. The organic layer was washed with a 10% ammonium chloride aqueous solution and a saturated saline, then dehydrated over anhydrous sodium sulfate, and concentrated under reduced pressure to obtain ethyl 2-(3,5-dichlorophenyl)-1,3-benzoxazole-6-carboxylate (360 mg, 1.07 mmol, yield: 83%) as a light yellow solid. The obtained spectral data were consistent with the spectral data described in (3) of Example 1.

Subsequently, the same procedure as in (4) of Example 1 was performed to obtain tafamidis in a yield of 93% (total yield from 4-amino-3-iodobenzoic acid: 34%) as a slightly yellowish white solid. The obtained spectral data were consistent with the spectral data described in (4) of Example 1.

### Example 8 (Synthesis of tafamidis meglumine)

2-Propanol (20 mL), water (5.5 mL), and N-methyl-D-glucamine (0.63 g, 3.25 mmol) were added to tafamidis (1.00 g, 3.25 mmol) obtained in Example 1, followed by stirring at an outside temperature of 80°C for 1 hour. Then, the mixture solution was cooled to an inside temperature of 10°C, and the precipitated solid was collected by filtration. The solid collected by filtration was dried under reduced pressure at an outside temperature of 50°C to obtain tafamidis meglumine (1.26 g, 2.50 mmol, yield: 77%) as a white solid.
¹H-NMR (DMSO): δ/ppm = 2.55 (s, 3H), 2.91-3.06 (m, 2H), 3.41-3.55 (m, 3H), 3.60 (dd, 1H, J = 3.2, 10.4 Hz), 3.71 (dd, 1H, J = 1.2, 4.8 Hz), 3.94 (m, 1H), 7.77 (d, 1H, J = 8.4 Hz), 7.92 (t, 1H, J = 2.0 Hz), 8.03 (dd, 1H, J = 1.2,8.4 Hz), 8.15 (d, 2H, J = 2.0 Hz), 8.19 (s, 1H)

### Comparative Example 1 (Synthesis of tafamidis)

Tafamidis was synthesized according to the method described in JP-A-2006-511612.
(1) That is, 4-amino-3-hydroxybenzoic acid (2.00 g, 13.1 mmol), pyridine (3.10 g, 39.2 mmol), and 3,5-dichlorobenzoyl chloride (2.74 g, 13.1 mmol) were added to tetrahydrofuran (200 mL), followed by reflux for 1 hour. The reaction mixture was concentrated under reduced pressure. The obtained N-[2-hydroxy-4-carboxyphenyl]-3,5-dichlorobenzamide was used in the subsequent step without being purified.
(2) Xylene (330 mL) and p-toluenesulfonic acid 1-hydrate (24.9 g, 131 mmol) were added to the concentrate of N-[2-hydroxy-4-carboxyphenyl]-3,5-dichlorobenzamide obtained above, followed by stirring at an outside temperature of 140°C for 14 hours. The reaction solution was cooled, and a 1 mol/L sodium hydroxide aqueous solution (131 mL, 131 mmol) was then added thereto to separate the organic layer. To the aqueous layer, 1 mol/L hydrochloric acid was added to adjust the pH to 2, followed by extraction with ethyl acetate (200 mL) four times. The extract was combined with the organic layer, followed by dehydration with anhydrous sulfonic acid and concentration under reduced pressure to obtain tafamidis (2.31 g, 7.5 mmol, yield: 57.2%) as a reddish white solid. In the method of Comparative Example 1, the yield was insufficient.

### Test Example 1

The maximum liquid volume (mL) in each step of the methods of Examples 1 to 7 and Comparative Example 1 for obtaining 1 g of tafamidis was estimated. In the halogenation step and the cyclization step, since the liquid volume leaches the maximum immediately before liquid separation, the liquid volume immediately before liquid separation was estimated as the maximum liquid volume. In the esterification step, the amidation step, and the hydrolysis step, since the liquid volume reaches the maximum immediately before filtration, the liquid volume immediately before filtration was estimated as the maximum liquid volume. In the estimate, the specific gravity of a liquid was regarded as 1 g/mL, and a solid was regarded as a liquid with a specific gravity of 1 g/mL.

Among the maximum liquid volumes in each step for obtaining 1 g of tafamidis, the largest liquid volume was recorded as the "maximum value".

It can be said that the smaller the maximum liquid volume in each step or the above "maximum value" for obtaining 1 g of tafamidis is, the easier it is to reduce the size of the production facilities necessary for producing tafamidis. The results are shown in Table 1.

The method for approximating the maximum liquid volume (mL) in each step for obtaining 1 g of tafamidis will be explained below by taking Example 1 as an example. In Example 1, among the halogenation step, the amidation step, the cyclization step, and the hydrolysis step, the largest liquid volume was 52 mL in the hydrolysis step. Accordingly, among the maximum liquid volumes in each step, the "maximum value" was recorded as 52 mL.

The total liquid volume in the hydrolysis step of Example 1 is approximately 164.49 mL. The details thereof are: tetrahydrofuran 64 mL; water 16 mL; ethyl 2-(3,5-dichlorophenyl)-1,3-benzoxazole-6-carboxylate about 3.99 mL; lithium hydroxide monohydrate about 0.70 mL; water 48 mL; and 1 mol/L hydrochloric acid 32 mL.

In this hydrolysis step, 3.17 g of tafamidis is obtained, and the maximum liquid volume in the hydrolysis step of Example 1 for obtaining 1 g of tafamidis is 164.49 ÷ 3.17 ≈ 52 mL.

The total liquid volume in the cyclization step of Example 1 is approximately 145.48 mL. The details thereof are: toluene 50 mL; N,N,N',N'-tetramethylethylene diamine about 0.28 mL; copper(I) iodide about 0.23 mL; N-[2-bromo-4-(ethoxycarbonyl)phenyl]-3,5-dichlorobenzamide about 5.00 mL; potassium carbonate about 4.97 mL; a 10% ammonium chloride aqueous solution 25 mL; and tetrahydrofuran 60 mL.

The amount of ethyl 2-(3,5-dichlorophenyl)-1,3-benzoxazole-6-carboxylate obtained in this cyclization step is 3.99 g, and the amount of ethyl 2-(3,5-dichlorophenyl)-1,3-benzoxazole-6-carboxylate necessary for obtaining 1 g of tafamidis is 3.99 ÷ 3.17 ≈ 1.26 g.

Accordingly, the maximum liquid volume in the cyclization step of Example 1 for obtaining 1 g of tafamidis is 145.48 ÷ 3.99 × 1.26 ≈ 46 mL.

The total liquid volume in the amidation step of Example 1 is approximately 7,690 mL. The details thereof are: ethyl 4-amino-3-bromobenzoate about 290 mL; tetrahydrofuran 2,900 mL; pyridine about 141 mL; tetrahydrofuran 580 mL; 3,5-dichlorobenzoyl chloride about 299 mL; and water 3,480 mL.

The amount of N-[2-bromo-4-(ethoxycarbonyl)phenyl]-3,5-dichlorobenzamide obtained in this amidation step is 460 g, and the amount of N-[2-bromo-4-(ethoxycarbonyl)phenyl]-3,5-dichlorobenzamide necessary for obtaining 1 g of tafamidis is 5.00 ÷ 3.99 × 1.26 ≈ 1.58 g.

Accordingly, the maximum liquid volume in the amidation step of Example 1 for obtaining 1 g of tafamidis is 7,690 ÷ 460 × 1.58 ≈ 26 mL.

The total liquid volume in the halogenation step of Example 1 is approximately 3,418 mL. The details thereof are: ethyl 4-aminobenzoate about 200 mL; tetrahydrofuran 1,000 mL; N-bromosuccinimide about 218 mL; water 1,000 mL; and ethyl acetate 1,000 mL.

The amount of ethyl 4-amino-3-bromobenzoate obtained in this halogenation step is 297 g, and the amount of ethyl 4-amino-3-bromobenzoate necessary for obtaining 1 g of tafamidis is 290 ÷ 460 × 1.58 ≈ 0.99 g.

Accordingly, the maximum liquid volume in the halogenation step of Example 1 for obtaining 1 g of tafamidis is 3,418 ÷ 297 × 0.99 ≈ 11 mL.

**[Table 1]**

| | Maximum liquid volume in each step for obtaining 1 g of tafamidis (mL) | | | | | Maximum value (mL) |
|---|---|---|---|---|---|---|
| | Halogenation step | Esterification step | Amidation step | Cyclization step | Hydrolysis step | |
| Example 1 | 11 | - | 26 | 46 | 52 | 52 (hydrolysis step) |
| Example 2 | 12 | - | 27 | 47 | 52 | 52 (hydrolysis step) |
| Example 3 | 12 | - | 27 | 47 | 52 | 52 (hydrolysis step) |
| Example 4 | 11 | - | 26 | 45 | 50 | 50 (hydrolysis step) |
| Example 5 | 14 | - | 33 | 57 | 46 | 57 (cyclization step) |
| Example 6 | 20 | - | 20 | 38 | 48 | 48 (hydrolysis step) |
| Example 7 | - | 29 | 60 | 57 | 48 | 60 (amidation step) |
| Comparative Example 1 | - | - | 90 | 211 | - | 211 (cyclization step) |

As shown in Table 1, the size of the production facilities necessary for producing tafamidis can be reduced by producing the target compound by the methods of Examples 1 to 7.

### Comparative Example 2 (Synthesis of ethyl 2-(3,5-dichlorophenyl)-1,3-benzoxazole-6-carboxylate)

N-[2-Bromo-4-(ethoxycarbonyl)phenyl]-3,5-dichlorobenzamide (4 g, 9.6 mmol), bis(1,5-cyclooctadiene)diiridium(I) dichloride (0.064 g, 0.12 mmol), and potassium acetate (2.82 g, 29 mmol) were added to dimethyl sulfoxide (12 mL), followed by stirring at an outside temperature of 100°C for 48 hours. The reaction conversion rate on this occasion was 51%. Water (20 mL) was added to this reaction solution, followed by extraction with a mixture solution of ethyl acetate (60 mL) and tetrahydrofuran (20 mL). The organic layer was washed with a saturated saline, then dehydrated over anhydrous sodium sulfate, and concentrated under reduced pressure. The concentrate was purified by column chromatography to obtain ethyl 2-(3,5-dichlorophenyl)-1,3-benzoxazole-6-carboxylate (1.08 g, 2.6 mmol, yield: 34%) as a light yellow solid. In the method of Comparative Example 2, the yield was insufficient.

As described above, when the compound (1) was cyclized in the presence of bis(1,5-cyclooctadiene)diiridium(I) dichloride (Comparative Example 2), the yield in the cyclization step was 34%.

In contrast, when the compound (1) was cyclized in the presence of one or more copper catalysts selected from the group consisting of copper halides, copper carboxylates, copper sulfates, and copper oxides, a base, and an amine ligand (Examples 1 to 7), the yield in the cyclization step was 71% to 100%. Here, it has been believed that an iridium catalyst is useful than a copper catalyst such as a copper iodide catalyst in order to improve the efficiency of cyclization by C-O coupling reaction (Tetrahedron Letters, 2019, 60, 151082). However, when one or more copper catalysts selected from the group consisting of copper halides, copper carboxylates, copper sulfates, and copper oxides, a base, and amine ligand are used, it is surprisingly that a large improvement in the yield was observed as described above.

### Reference Example 1 (Synthesis of N-[2-hydroxy-4-carboxyphenyl]-3,5-dichlorobenzamide)

N-[2-Hydroxy-4-carboxyphenyl]-3,5-dichlorobenzamide was synthesized with reference to the amidation reaction described in JP-A-2021-517118.

That is, 4-amino-3-hydroxybenzoic acid (2.00 g, 13.1 mmol) and pyridine (1.20 g, 15.2 mmol) were added to tetrahydrofuran (74 mL), followed by cooling to -12°C in an ice salt bath. 3,5-Dichlorobenzoyl chloride (2.68 g, 12.8 mmol) dissolved in tetrahydrofuran (12 mL) was added thereto, followed by changing the temperature to room temperature and stirring for 40 minutes. To the reaction solution, 0.2 mol/L hydrochloric acid (258 mL) was added. The precipitate was collected by filtration and dried under reduced pressure at an outside temperature of 50°C. To the obtained solid, a 0.5 mol/L sodium hydroxide aqueous solution (98 mL) was added, followed by stirring at room temperature. Insoluble substances were removed by filtration. The solution was washed with dichloromethane (48 mL), and 1 mol/L hydrochloric acid was added to the aqueous layer to adjust the pH to 2 to 3. the precipitate was collected by filtration and was dried under reduced pressure at an outside temperature of 50°C. 1-Butanol (122 mL) was added to the obtained solid, followed by reflux with heating at an outside temperature of 120°C for 7 hours and then stirring overnight at an outside temperature of 70°C. The solid was collected by filtration, and the obtained solid was dried under reduced pressure at an outside temperature of 50°C to obtain N-[2-hydroxy-4-carboxyphenyl]-3,5-dichlorobenzamide (2.64 g, 8.1 mmol, yield: 61.8%) as a light yellow solid.

### Reference Example 2 (Synthesis of N-[2-hydroxy-4-carboxyphenyl]-3,5-dichlorobenzamide)

N-[2-Hydroxy-4-carboxyphenyl]-3,5-dichlorobenzamide was synthesized with reference to the amidation reaction described in JP-A-2021-517118.

That is, 4-amino-3-hydroxybenzoic acid (2.00 g, 13.1 mmol) was dissolved in a mixture solution of tetrahydrofuran (36 mL) and water (4 mL), and 3,5-dichlorobenzoyl chloride (3.28 g, 15.7 mmol) was added thereto, followed by stirring at room temperature for 1 hour. Triethylamine (1.59 g, 15.7 mmol) was added to this mixture, followed by stirring at room temperature for 2 hours, and 0.1 mol/L hydrochloric acid (300 mL) was added thereto. The precipitate was collected by filtration, and a 0.5 mol/L sodium hydroxide aqueous solution was added to the obtained solid, followed by stirring at room temperature. Insoluble substances were removed by filtration. The solution was washed with dichloromethane (100 mL), and 1 mol/L hydrochloric acid (30 mL) and acetonitrile (130 mL) were added to the aqueous layer. The precipitate was collected by filtration and was dried under reduced pressure at an outside temperature of 50°C to obtain N-[2-hydroxy-4-carboxyphenyl]-3,5-dichlorobenzamide as a grayish white solid. The yield was about 65% as described in JP-A-2021-517118.

## Claims

1. A method for producing tafamidis or a salt thereof, comprising a cyclization step of cyclizing a compound of a formula (1) below in the presence of one or more copper catalysts selected from the group consisting of copper halides, copper carboxylates, copper sulfates, and copper oxides, a base, and an amine ligand to obtain a compound of a formula (2) below, 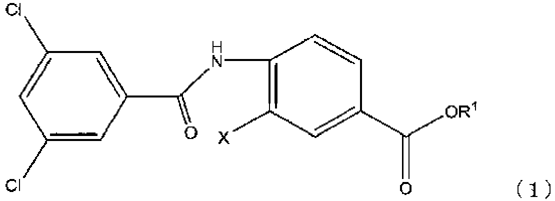 [in the formula (1),
R¹ represents a linear or branched alkyl group, and
X represents a halogen atom] 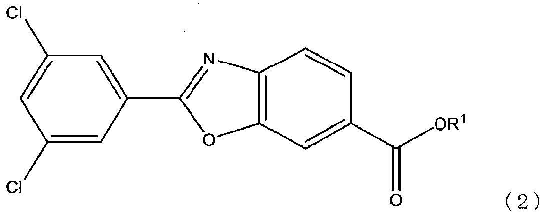
[in the formula (2), R¹ is as defined above].

2. The producing method according to Claim 1, further comprising an amidation step of amidating a compound of a formula (3) below and a compound of a formula (4) below in the presence of a base, to use the compound obtained in this step as the compound of the formula (1) in the cyclization step, 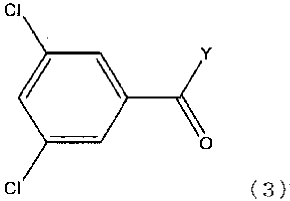 [in the formula (3), Y represents a halogen atom] 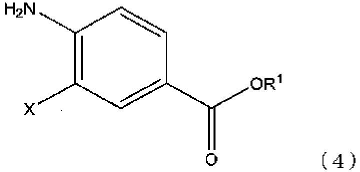 [in the formula (4), R¹ and X are as defined above].

3. The producing method according to Claim 2, further comprising a halogenation step of halogenating a compound of a formula (5) below, to use the compound obtained in this step as the compound of the formula (4) in the amidation step, 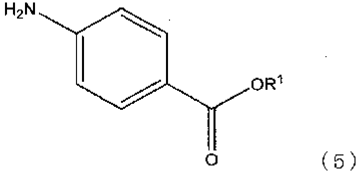 [in the formula (5), R¹ is as defined above].

4. The producing method according to Claim 2, further comprising an esterification step of subjecting a compound of a formula (11) below and a compound of a formula (12) below to dehydration-condensation reaction, to use the compound obtained in this step as the compound of the formula (4) in the amidation step, 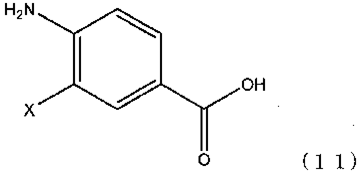 [in the formula (11), X is as defined above]
R¹OH (12)
[in the formula (12), R¹ is as defined above].

5. The producing method according to any one of Claims 1 to 4, further comprising a hydrolysis step of ester-hydrolyzing the compound of the formula (2) obtained in the cyclization step.

6. The producing method according to any one of Claims 1 to 5, wherein the copper catalyst to be used is one or more selected from the group consisting of copper(I) iodide, copper(II) iodide, copper(I) bromide, copper(II) bromide, copper(I) chloride, copper(II) chloride, copper(I) acetate, copper(II) acetate, copper trifluoroacetate, copper pentafluoropropionate, copper oxalate, copper(I) sulfate, copper(II) sulfate, copper(I) oxide, and copper(II) oxide.

7. The producing method according to any one of Claims 1 to 5, wherein the copper catalyst to be used is one or more copper catalysts selected from the group consisting of copper halides, copper carboxylates, and copper sulfates.

8. The producing method according to any one of Claims 1 to 5, wherein the copper catalyst to be used is a copper halide.

9. The producing method according to Claim 8, wherein the copper halide to be used is one or more copper halides selected from the group consisting of copper(I) iodide, copper(I) bromide, and copper(I) chloride.

10. The producing method according to any one of Claims 1 to 9, wherein the amine ligand to be used is an amine ligand selected from the group consisting of monovalent amine ligands and diamine ligands.

11. The producing method according to any one of Claims 1 to 10, wherein X is a chlorine atom or a bromine atom.
